# EUROPÄISCHE PATENTSCHRIFT

(11) **EP 1 059 519 B1**
(45) Veröffentlichungstag und Bekanntmachung des Hinweises auf die Patenterteilung: **02.11.2005**
(21) Anmeldenummer: 99111313.5
(22) Anmeldetag: 10.06.1999
(51) Int. Cl.: G01N 1/14, G01N 33/18

(54) **Probenehmer und Verfahren zum Abfüllen und Abkühlen eines Fluids**
Sampler and method of dosing and cooling a fluid
Echantilleur et procédé pour remplir et refroidir un fluide

(43) Veröffentlichungstag der Anmeldung: 13.12.2000
(73) Patentinhaber: ENDRESS + HAUSER WETZER GmbH + Co. KG, D-87484 Nesselwang (DE)
(72) Erfinder: Zeller, Robert, 87439 Kempten (DE)
(74) Vertreter: Andres, Angelika Maria

(56) Entgegenhaltungen:
- FR-A- 2 647 213
- US-A- 3 795 347
- US-A- 3 880 011
- US-A- 3 897 687
- US-A- 4 077 263
- US-A- 5 587 926

## Beschreibung

Die Erfindung betrifft einen Probenehmer und ein Verfahren zum Abfüllen und Abkühlen eines Fluids.

Fluide unterschiedlichster Herkunft und Anwendung sind häufig auf ihren chemisch-biologischen Zustand hin zu überwachen, der durch im Fluid mitgeführte Substanzen bestimmt wird. Zur Überwachung von wäßrigen Fluiden, insb. in der Trinkwasseraufbereitung oder in der Abwasserreinigung, sind dazu an entsprechenden Fluid-Entnahmestellen räumlich und zeitlich verteilt, repräsentative Fluid-Proben zu entnehmen und entsprechend zu untersuchen. Das Volumen derartiger Fluid-Proben liegt üblicherweise zwischen ca. 10 ml und 500 ml.

Die Entnahme und das Abfüllen erfolgt üblicherweise mittels eines Probenehmers. So ist in der US-A 37 95 347 z.B. ein Probenehmer zum Abfüllen einer Fluid-Probe eines an einer Fluid-Entnahmestelle entnommenen Fluids beschrieben, der umfaßt:
- eine Gefäßanordnung
   -- mit einem rohrförmigen Entnahmegefäß zum Führen eines strömenden Fluids,
   -- mit einem auslaßseits temporär verschließbaren Dosiergefäß zum Abdosieren der Fluid-Probe vom entnommenen Fluid,
   -- mit einem Verteilergefäß zum Weiterleiten der Fluid-Probe und
   -- mit einem Speichergefäß zum Speichern der Fluid-Probe.

Innerhalb des Fluid laufen aufgrund der mitgeführten Substanzen, insb. aufgrund von Bakterien, aber auch aufgrund bestimmter chemischer Verbindungen, Stoffwechselvorgänge ab, die den chemisch-biologischen Zustand des Fluids praktisch ständig verändern. Diese Änderung des chemisch-biologischen Zustand des Fluids je Zeiteinheit wird üblicherweise als Aktivität des Fluids bezeichnet. Die Aktivität ist temperaturabhängig und praktisch umso größer, je höher die Temperatur des Fluids ist.

Die Güte der Überwachung wird u.a. dadurch bestimmt, wie gut der chemisch-biologische Zustand der Fluid-Probe zum Zeitpunkt der Untersuchung mit dem chemisch-biologischen Zustand des Fluids zum Zeitpunkt der Entnahme übereinstimmt. Entnahme und Untersuchung der Fluid-Probe finden jedoch häufig über einen längeren Zeitraum voneinander getrennt statt. Daher werden die Fluid-Proben in geeignete Speichergefäße, z.B. Probeflaschen, abdosiert und in diesen eingelagert.

Soll die Fluid-Probe bei der Untersuchung den zum Zeitpunkt der Entnahme im Fluid vorhandenen chemisch-biologischen Zustand möglichst genau repräsentieren, so muß die über den Zeitraum zwischen Entnahme und Untersuchung gemittelte Aktivität der Fluid-Probe entsprechend minimiert werden. Üblicherweise werden die Fluid-Proben daher auf eine stationäre Lagerungs-Temperatur, von z.B. 4 °C (= 277 K), abgekühlt, bei der eine zulässige Aktivität nicht überschritten wird.

Zum Abkühlen der Fluid-Proben werden die Speichergefäße in einem Kühlraum bei einer entsprechenden Lagerungs-Temperatur untergebracht und die Fluid-Proben mittels des Probenehmers von der Fluid-Entnahmestelle praktisch direkt in die Speichergefäße abgefüllt und dort abgekühlt. Ein derartiges Kühlsystem ist z.B. aus der Schrift US-A-5 587 926 bekannt.

Es hat sich jedoch gezeigt, daß bei diesem Verfahren die Abkühlgeschwindigkeiten, insb. bei einer Fluid-Temperatur an der Fluid-Entnahmestelle von über 15 °C, zu gering und damit die Aktivität der einzulagernden Fluid-Probe bis zur Untersuchung zu groß sein können. So wurden bei einer Fluid-Temperatur von 16 °C und einem Volumen der Fluid-Probe von 500 ml eine mittlere Abkühlgeschwindigkeit von z.B. 1 K/h und damit eine Abkühlzeit bis zum Erreichen der Lagerungs-Temperatur von 12 h gemessen. Aufgrund der zu großen Aktivität der Fluid-Probe kann sich der chemisch-biologische Zustand der Fluid-Probe während des Abfüllens und Abkühlens erheblich im Vergleich zum momentanen chemisch-biologischen Zustand bei der Entnahme verändern.

Eine Aufgabe der Erfindung besteht darin, einen Probenehmer zum Abfüllen und Abkühlen einer solchen Fluid-Probe anzugeben, mit dem die Abkühlzeit und damit die Aktivität der Fluid-Probe reduziert werden kann.

Eine weitere Aufgabe der Erfindung ist es, ein entsprechendes Verfahren zum Abfüllen und Abkühlen einer derartigen Fluid-Probe anzugeben, dessen Anwendung eine Reduzierung der Abkühlzeit bewirkt.

Zur Lösung der Aufgabe besteht die Erfindung in einem Probenehmer zum Abfüllen und Abkühlen einer bei einer vorgebbaren Lagerungs-Temperatur einzulagernden Fluid-Probe eines an einer Fluid-Entnahmestelle entnommenen Fluids, das eine momentane Entnahme-Temperatur aufweist, die größer als die Lagerungs-Temperatur ist, welcher Probenehmer umfaßt:
- eine Gefäßanordnung von vorgebbarem Lumen mit
   -- einer über das Lumen räumlich gemittelten Gefäßanordnungs-Innentemperatur
   -- einem rohrförmigen Entnahmegefäß zum Führen eines strömenden Fluids,
   -- einem auslaßseits temporär verschließbaren Dosiergefäß zum Abdosieren der Fluid-Probe vom entnommenen Fluid,
   -- einem Verteilergefäß zum Weiterleiten der Fluid-Probe und
   -- einem Speichergefäß zum Speichern der Fluid-Probe sowie
- eine mit der Gefäßanordnung thermisch gekoppelte Kühlanordnung zum Einstellen der Gefäßanordnungs-Innentemperatur, mit
   -- einem wenigstens das Speichergefäß umhüllenden ersten Kühlvolumen von vorgebbarer erster Kühltemperatur zum Kühlen der Fluid-Probe auf die Lagerungs-Temperatur und
   -- einem die Gefäßanordnung wenigstens abschnittsweise ummantelnden zweiten Kühlvolumen von vorgebbarer zweiter Kühltemperatur zum Kühlen des entnommenen Fluids unter die Fluid-Entnahmetemperatur.

Ferner besteht die Erfindung in einem Verfahren zum Abfüllen und Abkühlen einer bei einer vorgebbaren Lagerungs-Temperatur einzulagernden Fluid-Probe eines an einer Fluid-Entnahmestelle (1) entnommenen Fluids, das eine momentane Entnahme-Temperatur aufweist, die größer als die Lagerungs-Temperatur ist, mittels eines Probenehmers, der umfaßt:
- eine Gefäßanordnung von vorgebbarem Lumen mit
   -- einer über das Lumen räumlich gemittelten Gefäßanordnungs-Innentemperatur
   -- einem rohrförmigen Entnahmegefäß zum Führen eines strömenden Fluids,
   -- einem auslaßseits temporär verschließbaren Dosiergefäß zum Abdosieren der Fluid-Probe vom entnommenen Fluid,
   -- einem Verteilergefäß zum Weiterleiten der Fluid-Probe,
   -- mit einem Speichergefäß zum Speichern der Fluid-Probe, und
- eine mit der Gefäßanordnung thermisch gekoppelte Kühlanordnung zum Einstellen der Gefäßanordnungs-Innentemperatur mit
   -- einem wenigstens das Speichergefäß umhüllenden ersten Kühlvolumen von vorgebbarer erster Kühltemperatur zum Kühlen der Fluid-Probe auf die Lagerungs-Temperatur, wobei
- die Gefäßanordnungs-Innentemperatur vor dem Abfüllen eine Anfangs-Gefäßanordnungs-Innentemperatur aufweist, die niedriger als die Entnahme-Temperatur ist, und
- die erste Kühltemperatur vor dem Abfüllen und nach dem Einlagern der Fluid-Probe auf die Lagerungs-Temperatur eingestellt ist,
welches Verfahren folgende Schritte umfaßt:
- die Fluid-Probe wird dadurch abgefüllt, daß
   -- das Fluid von der Fluid-Entnahmestelle aus durch das Entnahmegefäß hindurch in das auslaßseits verschlossene Dosiergefäß gefördert wird,
   -- ein vorgebbares Teil-Volumen des Fluids im Dosiergefäß belassen wird,
   -- das Dosiergefäß auslaßseits geöffnet wird und
   -- das Teil-Volumen des Fluids durch das Verteilergefäß hindurch in das Speichergefäß einströmen gelassen wird, und
- die Fluid-Probe wird dadurch abgekühlt, daß
   -- die Gefäßanordnungs-Innentemperatur mittels der Kühlanordnung vor und/oder während des Abfüllens auf eine Abkühlungs-Gefäßanordnungs-Innentemperatur abgesenkt wird, die niedriger als die Anfangs-Gefäßanordnungs-Innentemperatur ist, und
   -- das Fluid nach dem Einstellen der Abkühlungs-Gefäßanordnungs-Innentemperatur in der Gefäßanordnung (2) geführt und/oder gehalten wird.

Nach einer ersten Ausgestaltung der Erfindung ist das zweite Kühlvolumen vom ersten Kühlvolumen wenigstens teilweise umhüllt.

Nach einer zweiten Ausgestaltung der Erfindung umfaßt die Kühlanordnung
- ein am Entnahmegefäß angeordnetes erstes Kühlelement zum Einstellen einer Entnahmegefäß-Innentemperatur und/oder
- ein am Dosiergefäß angeordnetes zweites Kühlelement zum Einstellen einer Dosiergefäß-Innentemperatur und/oder
- ein am Verteilergefäß angeordnetes drittes Kühlelement zum Einstellen einer Speichergefäß-Innentemperatur und/oder
- ein am Speichergefäß angeordnetes viertes Kühlelement zum Einstellen der Speichergefäß-Innentemperatur,
- wobei das jeweilige Kühlelement das zweite Kühlvolumen abschnittsweise ummantelt.

Nach einer dritten Ausgestaltung der Erfindung ist das erste Kühlelement ein Durchlaufkühler.

Nach einer vierten Ausgestaltung der Erfindung umfaßt wenigstens eines der Kühlelemente ein Peltier-Element.

Nach einer fünften Ausgestaltung der Erfindung ist das Dosiergefäß innerhalb des ersten Kühlvolumens angeordnet.

Nach einer sechsten Ausgestaltung der Erfindung ist das Entnahmegefäß vom ersten Kühlvolumen teilweise umhüllt.

Nach einer siebenten Ausgestaltung der Erfindung umfaßt die Kühlanordnung einen Kühlraum, dessen Lumen das erste Kühlvolumen ist.

Nach einer achten Ausgestaltung der Erfindung ist das Fluid ein Trinkwasser oder ein Abwasser.

Nach einer neunten Ausgestaltung der Erfindung wird die Gefäßanordnungs-Innentemperatur dadurch auf die Abkühlungs-Gefäßanordnungs-Innentemperatur eingestellt, daß die erste Kühltemperatur temporär auf eine erste Abkühlungs-Temperatur abgesenkt wird, die niedriger als die Lagerungs-Temperatur ist.

Nach einer zehnten Ausgestaltung der Erfindung
- wird eine Kühlanordnung mit einem die Gefäßanordnung wenigstens abschnittsweise umgebenden zweiten Kühlvolumen von vorgebbarer zweiter Kühltemperatur zum Kühlen des entnommenen Fluids unter die Entnahme-Temperatur verwendet und
- wird die Abkühlungs-Gefäßanordnungs-Innentemperatur vor dem Einströmen des Fluids in das Dosiergefäß durch ein temporäres Einstellen der zweiten Kühltemperatur auf eine zweite Abkühlungs-Temperatur, die niedriger als die Lagerungs-Temperatur ist, eingestellt.

Nach einer elften Ausgestaltung der Erfindung wird die Fluid-Probe von einem wäßriges Fluid entnommen.

Ein Grundgedanke des Verfahrens der Erfindung besteht darin, die Aktivität des entnommenen Fluids und damit auch die der Fluid-Probe bereits während des Abfüllens abzusenken und in möglichst kurzer Zeit ein gefordertes Minimum der Aktivität zu erreichen. Dies wird bei der Erfindung dadurch erreicht, daß ein fluidführendes Lumen der Gefäßanordnung vor dem Abfüllen mittels einer entsprechenden Kühlanordnung abgekühlt wird. Dieses abkühlbare Lumen kann sich dabei über ein Gesamt-Innenvolumen der Gefäßanordnung erstrecken, wodurch die Abkühlung des Fluids unmittelbar mit dessen Einströmen in die Gefäßanordnung beginnt, oder nur einen Teil des Gesamt-Innenvolumens umfassen.

Ein Vorteil des Verfahrens der Erfindung ist, daß die Aktivität der Fluid-Probe, insb. auch bei höheren Fluid-Temperaturen an der Fluid-Entnahmestelle, sehr rasch absenkbar ist. Ein weiterer Vorteil des Verfahrens besteht darin, daß es auch bei bereits bestehenden Gefäßanordnungen anwendbar ist.

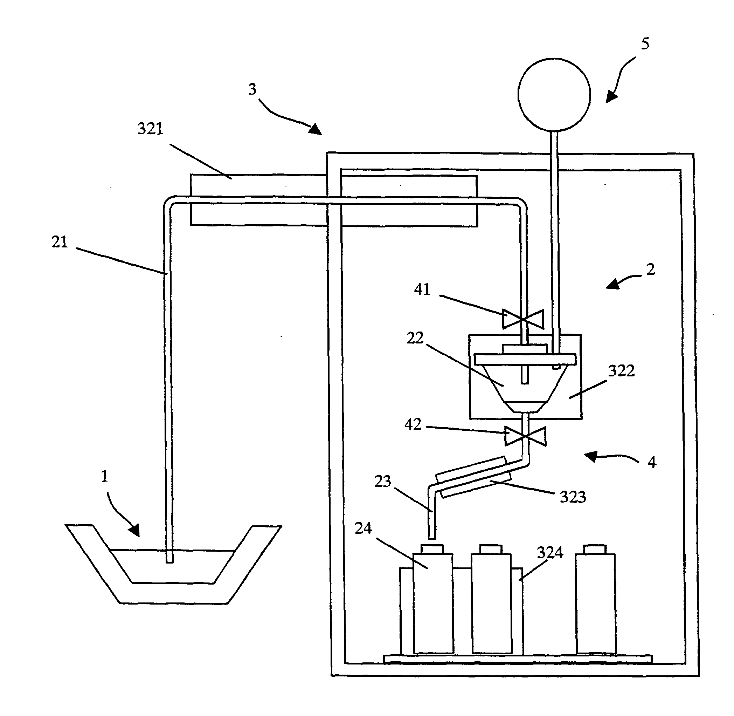

Die Erfindung wird nun anhand der einzigen Figur der Zeichnung näher erläutert, in der ein Ausführungsbeispiel der Erfindung dargestellt ist.

In der Figur ist ein Probenehmer zum Entnehmen eines, insb. wäßriges, Fluids von einer Fluid-Entnahmestelle 1 und zum Abdosieren, Abfüllen und Speichern einer Fluid-Probe des entnommenen Fluids schematisch dargestellt. Dieser Probenehmer umfaßt dazu eine Gefäßanordnung 2 von vorgebbarem Lumen, die dem Führen und Halten des Fluids bzw. der Fluid-Probe dient.

Der Probenehmer kann sowohl ein ortsfester als auch ein mobiler Probenehmer sein.

Die Gefäßanordnung 2 des Probenehmers umfaßt gemäß der Figur ein Entnahmegefäß 21 zum Entnehmen und Führen eines ersten Fluid-Volumens, ein Dosiergefäß 22 zum Aufnehmen eines zweiten Fluid-Volumens vom ersten Fluid-Volumen und zum Abdosieren eines dritten Fluid-Volumens vom zweiten Fluid-Volumen sowie ein Verteilergefäß 23 zum Führen des dritten Fluid-Volumens. Außerdem umfaßt die Gefäßanordnung 2 im Betrieb ein Speichergefäß 24 zum Speichern der einzulagernden Fluid-Probe vom dritten Fluid-Volumen. Jedes dieser Gefäße 21, 22, 23, 24 der Gefäßanordnung 2 ist durch eine umhüllende Wandung von vorgebbarer Form und Stärke festgelegt und dadurch gegenüber der Umgebung abgegrenzt.

Das Entnahmegefäß 21 ist rohrförmig ausgeführt und weist ein einlaßseitiges erstes und ein auslaßseitiges zweites Ende auf. Bevorzugt ist das Entnahmegefäß 21 wenigstens abschnittsweise als flexibler Schlauch, insb. von elastischem Material, ausgebildet. Als Material können alle in Probenehmer für derartige Entnahmegefäße üblichen Materialien, wie z.B. Polyethylen oder Glas, verwendet werden.

Das Entnahmegefäß 21 ist mit seinem zweiten Ende mit einer ersten Ein/Auslaß-Öffnung des Dosiergefäßes 22 verbunden, die sich, wie in der Figur gezeigt, bevorzugt an einem höchsten Punkt des Dosiergefäßes 22 bzw. in dessen Nähe befindet; falls erforderlich, kann die Ein/Auslaß-Öffnung auch an einem tiefer liegenden Punkt des Dosiergefäßes 22 angeordnet sein.

Das Dosiergefäß 22 weist bevorzugt ein von der Ein/Auslaß-Öffnung ausgehendes und mit einem Ende in dessen Lumen hineinragendes rohrförmiges Ein/Auslaß-Stück von vorgebbarer Länge auf. Für den Fall, daß die vorgebbare Länge des Ein/Auslaß-Stücks im Betrieb des Probenehmers nicht variierbar ist, ist das Ein/Auslaß-Stück üblicherweise durch einen auslaßseitigen Abschnitt des Entnahmegefäßes 21, der von oben senkrecht in das Lumen des Dosiergefäßes 22 hineinragt, realisiert.

Das Dosiergefäß 22 besteht bevorzugt aus Glas; es kann aber auch aus jedem anderen für derartige Dosiergefäße üblicherweise verwendeten Material, z.B. Polyethylen, bestehen.

An eine temporär verschließbare Auslaß-Öffnung des Dosiergefäßes 22 ist das Verteilergefäß 23 mit einem einlaßseitigen ersten Ende angeschlossen. Die Auslaß-Öffnung befindet sich nach der Figur bevorzugt an einem tiefsten Punkt des Dosiergefäßes 22; sie kann, falls erforderlich, auch an einem anderen Punkt des Dosiergefäßes 22 angeordnet sein.

Das Verteilergefäß 23 ist wie das Entnahmegefäß 21 ebenfalls rohrförmig, insb. abschnittsweise elastisch verformbar, ausgeführt. Es ist so geformt und dimensioniert, daß ein durch dessen auslaßseitiges Ende strömendes Fluid durch eine Einlaß-Öffnung des Speichergefäßes 24 in dieses einleitbar ist.

Für den Fall, daß das Verteilergefäß 23, wie in der Figur dargestellt, nur ein einziges auslaßseitiges Ende aufweist jedoch mittels des Probenehmers nacheinander mehrere Fluid-Proben abgefüllt werden sollen, ist das Verteilergefäß 23 bevorzugt verschwenkbar so ausgeführt, daß ein durch das zweite Ende strömendes Fluid in weitere, vom Speichergefäß 24 beabstandete Speichergefäße einleitbar ist. Das Verteilergefäß 23 kann aber auch mehrere auslaßseitige Enden aufweisen und so ausgeführt sein, daß bei mehreren zu befüllenden Speichergefäßen jedem eines der auslaßseitigen Enden so zugeordnet ist, daß das durch die Enden strömende Fluid in das jeweilige Speichergefäß einleitbar ist.

Bei dem mit dem Probenehmer zu entnehmenden Fluid handelt es sich um ein Fluid, insb. Trinkwasser oder Abwasser, das auf seine chemischen und/oder biologischen Eigenschaften, z.B. die mitgeführten Stoffe oder Bakterien, zu untersuchen ist. Derartige Fluide weisen eine temperaturabhängige Aktivität auf, die nach der Entnahme eine Veränderung dieser zu untersuchenden chemisch-biologischen Eigenschaften bewirken kann. Die Aktivität ist dabei umso größer, je höher eine Fluid-Temperatur. Daher wird das bei einer momentanen Entnahme-Temperatur T₁ entnommenen Fluid nach der Entnahme bei einer vorgebbaren niedrigen, insb. stationären, Lagerungs-Temperatur T_{L} eingelagert. Diese Lagerungs-Temperatur T_{L} ist so bemessen, daß die resultierende Aktivität soweit verringert ist, daß sie die Fluid-Probe nicht unzulässig verändert.

Üblicherweise erfolgt die Entnahme des Fluids zumeist bei einer relativ hohen Entnahme-Temperatur T₁, von z.B. 288 K (Kelvin), bei der das Fluid eine entsprechend große Aktivität aufweisen kann. Dies wiederum erfordert ein schnelles Abkühlen der Fluid-Probe, d.h. eine zwischen dem Beginn der Entnahme und dem Erreichen der Lagerungs-Temperatur T_{L} in der Fluid-Probe andauernde Abkühlungs-Phase Δta ist zu minimieren.

Der Probenehmer umfaßt daher des weiteren eine mit der Gefäßanordnung 2 thermisch gekoppelte Kühlanordnung 3 zum Kühlen des entnommenen Fluids. Thermisch gekoppelt heißt, daß zwischen der Kühlanordnung 3 und der Gefäßanordnung 2, insb. einem Lumen der Gefäßanordnung 2, temporär vorhandene Temperaturunterschiede nahezu ausgeglichen werden können.

Die Kühlanordnung 3 umschließt ein im Betrieb des Probenehmers wenigstens das Speichergefäß 24 umhüllendes erstes Kühlvolumen von vorgebbarer räumlich gemittelter erster Kühltemperatur T₃₁.

Das erste Kühlvolumen ist durch eine äußere, insb. thermostatisierte, Umhüllung umschlossen, und so nach außen hin abgrenzt. Die Umhüllung dient der Wärmeisolierung des ersten Kühlvolumens und üblicherweise auch als Trag- und Haltekonstruktion für die Gefäßanordnung 2. Sie besteht daher einerseits aus wärmeisolierendem Material und andererseits aus mechanisch festem Konstruktionsmaterial.

Das erste Kühlvolumen ist bei dem in der Figur dargestellten Ausführungsbeispiel durch das Lumen eines Kühlraums 311 gebildet. Dieser kann z.B. ein mobiler Kühlbehälter oder eine ortsfeste Kühlkammer sein.

Das erste Kühlvolumen ist bevorzugt so ausgeführt, daß es sich zusätzlich auch über das Dosiergefäß 22 und auch über einen Teil des Entnahmegefäßes 21 erstreckt. Ferner kann es so ausgeführt sein, daß darin neben dem Speichergefäß 24 gleichzeitig noch weitere Speichergefäße untergebracht werden können.

Gemäß der Figur umfaßt der Probenehmer des weiteren eine Absperr-Anordnung 4, die dem temporären und abschnittsweisen druckdichten Verschließen der Gefäßanordnung 2, insb. des Dosiergefäßes 22, dient.

Zum temporären Verschließen der Ein/Auslaß-Öffnung des Dosiergefäßes 22 ist an dieser bzw. am Entnahmegefäß 21 ein erstes Absperr-Element 41 der Absperr-Anordnung 4 angebracht. Des weiteren ist zum temporären Verschließen der Auslaß-Öffnung des Dosiergefäßes 22 an dieser bzw. am Verteilergefäß 23 ein zweites Absperr-Element 42 der Absperr-Anordnung 4 angebracht.

Als Absperr-Elemente 41, 42 können üblicherweise verwendete manuell, elektromechanisch oder pneumatisch angetriebenen Ventile bzw. Schieber dienen, vgl. die US-A 37 95 347 oder die US-A 38 80 011. Für den Fall, daß ein mindestens abschnittsweise elastisches Verteilergefäß 23 verwendet wird, ist das Absperr-Element bevorzugt als eine Schlauchklemme ausgebildet, vgl. die US-A 40 77 263.

Zu Beginn des Entnahmebetriebes des Probenehmers wird das erste Ende des Entnahmegefäßes 21, z.B. durch Eintauchen, so mit der Fluid-Entnahmestelle 1 verbunden, daß es, wie in der Figur dargestellt, mit dieser kommuniziert. Die Fluid-Entnahmestelle 1 kann sich dabei in jedem geeigneten Teil-Volumen des zu entnehmenden Fluids befinden.

In das mit der Fluid-Entnahmestelle 1 kommunizierende Entnahmegefäß 21 wird daraufhin das Fluid zu einem ersten Zeitpunkt t₁ einströmen gelassen und so darin weitergeleitet, daß es zu einem zweiten Zeitpunkt t₂ das an das Entnahmegefäß 21 angeschlossene Dosiergefäß 22 erreicht.

Wie in der Figur schematisch dargestellt, erfolgt dies dadurch, daß das Entnahmegefäß 21 in das, z.B. in einem offenen Gerinne oder Becken geführte, Fluid eingetaucht und entgegen der Schwerkraft abgezogen wird; es kann aber auch von einer geeigneten Fluid-Entnahmestelle 1 aus in Richtung der Schwerkraft und/oder aus einer Rohrleitung abgezogen werden.

Probenehmer der beschriebenen Art weisen daher üblicherweise eine an die Gefäßanordnung 2 angeschlossene Druck-Quelle 5 auf, die dem Erzeugen eines statischen Drucks von vorgebbarer Größe in einem Lumen der Gefäßanordnung 2, insb. im Lumen des Entnahmegefäßes 21 und des Dosiergefäßes 22, dient, vgl. auch die US-A 37 95 347, US-A 38 80 011 oder US-A 40 77 263.

Zum Entnehmen des ersten Fluid-Volumen wird im Lumen zwischen einlaßseitigem und auslaßseitigem Ende des mit der Fluid-Entnahmestelle 1 kommunizierende Entnahmegefäßes 21 eine erste Druckdifferenz erzeugt, die das Einströmen des Fluids in das Entnahmegefäß 21 und in das daran angeschlossene Dosiergefäß 22 bewirkt.

Beim Probenehmer gemäß der Figur wird dazu bei geöffneter Ein/Auslaß-Öffnung und bei geschlossener Auslaß-Öffnung im Lumen des Dosiergefäßes 22 mittels der Druck-Quelle 5 der statische Druck mindestens soweit reduziert, daß das daraufhin in das Entnahmegefäß 21 entgegen der Schwerkraft einströmende Fluid mindestens ein höchstes Niveau des Entnahmegefäßes 21 erreicht, vgl. auch die US-A 40 77 263.

Das Fluid wird solange in das Dosiergefäß 22 einströmen gelassen, bis dieses mit dem zweiten Fluid-Volumen befüllt ist. Dieses ist üblicherweise so bemessen, daß ein festlegbarer erster Füllstand im Dosiergefäß 22 erreicht wird, der sich oberhalb des Endes des Ein/Auslaß-Stückes befindet.

Nachdem das zweite Fluid-Volumen in das Dosiergefäß 22 eingefüllt ist, wird im zwischen dem Ende des Ein/Auslaß-Stücks und dem ersten Ende des Entnahmegefäßes 21 befindlichen Lumen der Gefäßanordnung 2 eine zweite Druckdifferenz derart erzeugt, daß ein Teil vom zweiten Fluid-Volumen durch das Ein/Auslaß-Stück hindurch wieder zurück in das Entnahmegefäß 21 und von dort weiter zur Fluid-Entnahmestelle 1 strömt. Das Zurückströmen des Fluids erfolgt genau so lange, bis ein zweiter Füllstand das Ende des Ein/Auslaß-Stück erreicht und sich somit im Dosiergefäß 22 nur noch das dritte Fluid-Volumen als ein Teil-Volumen des ersten Fluid-Volumen befindet.

Die zweite Druckdifferenz kann z.B. dadurch erzeugt werden, daß mittels der Druck-Quelle 5 der statische Druck im Dosiergefäß 22 mindestens soweit erhöht wird, daß das daraufhin in das Ein/Auslaß-Stück und in das Entnahmegefäß 21 entgegen der Schwerkraft einströmende Fluid mindestens das höchste Niveau des Entnahmegefäßes 21 erreicht.

Falls sich, wie in der Figur dargestellt, das Ende des Ein/Auslaß-Stücks auf einem höheren Niveau als ein Fluid-Pegel an der Fluid-Entnahmestelle 1 befindet und damit zwischen beiden eine Höhendifferenz besteht, wird diese Druckdifferenz bevorzugt dadurch erzeugt, daß zwischen der Fluid-Entnahmestelle 1 und dem Dosiergefäß 22 ein Ausgleich von deren statischen Drücken bewirkt wird. Dieser Ausgleich kann bei einer zur Atmosphäre hin geöffneten Fluid-Entnahmestelle 1 z.B. durch einfaches Belüften des Dosiergefäßes 22 erfolgen. Die resultierende Druckdifferenz wird dann im wesentlichen durch die zwischen dem Ende des Ein/Auslaß-Stücks und dem Fluid-Pegel bestehende Höhendifferenz bestimmt.

Nachdem das Dosiergefäß 22 nunmehr nur noch das dritte Fluid-Volumen faßt, wird zu einem dritten Zeitpunkt t₃ die Auslaß-Öffnung geöffnet und das dritte Fluid-Volumen mittels des Verteilergefäßes 23 in das Speichergefäß 24 abgefüllt bzw. bei der Verwendung mehrerer Speichergefäße in der eingangs beschriebenen Weise auf diese aufgeteilt.

Die im Speichergefäß 24 bzw. in den Speichergefäße befindliche Fluid-Probe wird nach dem Erreichen der im wesentlichen stationären Lagerungs-Temperatur T_{L}, mit einem Temperaturwert, von z.B. 277 K (Kelvin), zu einem Zeitpunkt t₄ eingelagert. Die Lagerungs-Temperatur T_{L} ist dabei höchstens gleich einer zulässigen höchsten Lagerungs-Temperatur des Fluids jedoch mindestens gleich einer zulässigen tiefsten Lagerungs-Temperatur des Fluids. Für den Fall, daß verschiedene Fluide im ersten Kühlvolumen untergebracht sind, ist die zulässige höchste Lagerungs-Temperatur gleich einer niedrigsten der jeweils zulässigen höchsten bzw. ist die zulässige tiefste Lagerungs-Temperatur gleich einer höchsten der jeweils zulässigen tiefsten Lagerungs-Temperatur der Fluide.

In entsprechender Weise ist die Kühltemperatur T₃₁ im Betrieb des ersten Kühlvolumens, insb. bei darin befindlichen Fluid-Proben, stets höchstens gleich der zulässigen höchsten Lagerungs-Temperatur eingestellt und somit praktisch auch stets niedriger als eine Umgebungs-Temperatur außerhalb des ersten Kühlvolumens. Die Kühltemperatur T₃₁ wird üblicherweise mittels einer mit dem ersten Kühlvolumen thermisch gekoppelten, jedoch nicht dargestellten aktiven Wärme-Senke, z.B. mittels einer Wärmepumpe oder eines Peltier-Element, eingestellt. Als Einstellverfahren können alle dem Fachmann bekannten Temperatursteuerungs- bzw. Temperaturregelungsverfahren dienen. Im Betrieb des Probenehmers liegt zu jedem Zeitpunkt im Lumen der Gefäßanordnung 2, insb. an und in deren Wandungen, jeweils eine momentane Innentemperatur-Verteilung mit einer über diese räumlich gemittelten Gefäßanordnungs-Innentemperatur T₂ vor. Die Höhe der Gefäßanordnungs-Innentemperatur T₂ ist aufgrund von Wärmeübergängen bzw. Wärmeleitung sowie aufgrund von Konvektion in der Gefäßanordnung 2 auch von der Höhe der Kühltemperatur T₃₁ abhängig.

Zu einem Anfangs-Zeitpunkt t₀ vor der Entnahme des Fluids ist die Kühltemperatur T₃₁ auf die Lagerungs-Temperatur T_{L} des Fluids eingestellt. Somit ist zum Anfangs-Zeitpunkt t₀ eine momentane Anfangs-Innentemperatur-Verteilung mit einer entsprechenden momentanen Anfangs-Gefäßanordnungs-Innentemperatur ausgebildet, die niedriger als die Entnahme-Temperatur T₁ ist.

Da das erste Kühlvolumen gemäß der Figur die Gefäßanordnung 2 nur teilweise umschließt, ist deren Innentemperatur-Verteilung, insb. bei einem außerhalb des ersten Kühlvolumens nicht- oder nur teilweise thermostatisiertem Entnahmegefäß 21 bzw. Dosiergefäß 22, durch eine äußere Umgebungstemperatur-Verteilung entsprechend mitbeeinflußt.

Je weiter sich das ersten Kühlvolumen 31 über die Gefäßanordnung 2 erstreckt und/oder je niedriger eine über die Abkühlungs-Phase Δta gemittelte erste Kühltemperatur T_{31, Δta} eingestellt ist, desto kleiner ist ein entsprechendes Temperatur-Verhältnis T_{2, Δta}/T_{L} von über die Abkühlungs-Phase Δta zeitlich gemittelter Gefäßanordnungs-Innentemperatur T_{2, Δta} zur Lagerungs-Temperatur T_{L}.

Je kleiner dieses Temperatur-Verhältnis T_{2, Δta}/T_{L} ist, desto größer ist im jeweiligen Probenehmer eine ebenfalls über der Abkühlungs-Phase Δta gemittelte Abkühlungs-Geschwindigkeit eines in der Gefäßanordnung 2 befindlichen Fluids. Eine Erhöhung der Abkühlungs-Geschwindigkeit verkürzt gleichzeitig die Zeit bis die Fluid-Probe die Lagerungs-Temperatur T_{L} erreicht hat und damit die Abkühlungs-Phase Δta. Die Abkühlungs-Phase Δta ergibt sich dabei aus der zeitlichen Differenz t₄-t₁, die vom Beginn der Entnahme zum Zeitpunkt t₁ bis zum Erreichen der Lagerungstemperatur in der Fluid-Probe zum Zeitpunkt t₄ andauert.

Gemäß dem Verfahren der Erfindung wird daher vor der Entnahme des ersten Fluid-Volumens die Gefäßanordnungs-Innentemperatur von der Anfangs-Gefäßanordnungs-Innentemperatur auf eine eine über die Gefäßanordnung 2 gemittelte, insb. zum Zeitpunkt t₁, niedrigere Abkühlungs-Gefäßanordnungs-Innentemperatur abgesenkt.

Nach einer Ausgestaltung der Erfindung wird die Kühltemperatur T₃₁ dazu mittels der aktiven Wärme-Senke von der Lagerungs-Temperatur T_{L} temporär auf eine Abkühlungs-Temperatur von z.B. 273 K (= 0 °C), abgesenkt. Dadurch ändert sich entsprechend auch die gesamte Innentemperatur-Verteilung im Lumen der Gefäßanordnung 2.

Während oder nach dem Absenken der Kühltemperatur T₃₁ wird das Fluid wie erwähnt in die Gefäßanordnung 2 einströmen gelassen. Das einströmende Fluid wird daraufhin aufgrund der innerhalb der Gefäßanordnung 2 bestehenden niedrigeren Abkühlungs-Gefäßanordnungs-Innentemperatur unter die Entnahme-Temperatur T₁ abgekühlt. Dies erfolgt im wesentlichen durch Wärmeübergang zu bzw. durch Wärmeleitung in der Wandung der Gefäßanordnung 2. Das Abkühlen des Fluids beginnt somit praktisch mit dem Einströmen in deren vom ersten Kühlvolumen 31 umschlossenen Teil und erfolgt, insb. im Speichergefäß 24, praktisch so lange, bis das Fluid eine stationäre Temperatur, insb. die Lagerungs-Temperatur T_{L}, angenommen hat, die einer stationären Innentemperatur-Verteilung entspricht.

Das Absenken und Wiederanheben der ersten Kühltemperatur T₃₁, und damit auch das Abkühlen des Fluids innerhalb der Gefäßanordnung 2, insb. innerhalb des Dosiergefäßes 22 bzw. des Speichergefäßes 24, kann geregelt und/oder zeitgesteuert erfolgen. Die entsprechenden Regelungs- bzw. Steuerungsgrößen sind durch entsprechende Kalibriermessungen ermittelbar.

Während der Abkühlungsphase Δta ist die Kühltemperatur T₃₁, falls erforderlich, auch auf eine niedrigere, unterhalb von 273 K liegende Abkühlungs-Temperatur einstellbar. Die Abkühlungs-Temperatur ist jedoch nur in soweit abzusenken, daß weder unzulässig hohe Abkühlungs-Geschwindigkeiten im Fluid erreicht noch bereits im ersten Kühlvolumen 31 eingelagerte Fluid-Proben unzulässig unterkühlt werden. Daher ist das Absenken bzw. das Wiederanheben der Kühltemperatur T₃₁ ggf. dadurch zu modifizieren, daß die Kühltemperatur T₃₁ während der Abkühlungs-Phase Δta auf Temperatur-Zwischenwerte eingestellt wird, die höher als die Abkühlungs-Temperatur sind. Die entsprechenden Temperatur-Zwischenwerte sind in geeigneter Weise ebenfalls durch entsprechende Kalibrierung ermittelbar.

Nach einer weiteren Ausgestaltung des Gegenstandes der Erfindung umfaßt die Kühlanordnung 3 ein die Gefäßanordnung 2 wenigstens teilweise umschließendes zweites Kühlvolumen von vorgebbarer räumlich gemittelter zweiter Kühltemperatur T₃₂, das insb. innerhalb der Wandung der Gefäßanordnung 2 zumindest abschnittsweise und im dadurch ummantelten Lumen der Gefäßanordnung 2 ausgebildet ist.

Das zweite Kühlvolumen dient einerseits der weiteren Ausdehnung des kühlbaren Lumens der Gefäßanordnung 2 in Richtung der Fluid-Entnahmestelle 1. Andererseits dient es der gezielten Einflußnahme auf die Innentemperatur-Verteilung der Gefäßanordnung 2 und damit einem feineren und genaueren Einstellen der Gefäßanordnungs-Innentemperatur T₂ während der Abkühlungs-Phase Δta.

Gemäß der Figur wird das zweite Kühlvolumen durch ein erstes Kühlelement 321 am Entnahmegefäß 21 zum Einstellen einer Entnahmegefäß-Innentemperatur, ein zweites Kühlelement 322 am Dosiergefäß 22 zum Einstellen einer Dosiergefäß-Innentemperatur, ein drittes Kühlelement 323 am Verteilergefäß 23 zum Einstellen einer Verteilergefäß-Innentemperatur und ein viertes Kühlelement 324 am Speichergefäß 24 zum Einstellen der Speichergefäß-Innentemperatur jeweils abschnittsweise umschlossen, und damit praktisch in entsprechende Teil-Kühlvolumen aufgeteilt.

Als Kühlelemente, insb. als Kühlelement 321, können wiederum entsprechende Wärmepumpen dienen. Nach einer bevorzugten Ausgestaltung der Erfindung ist das Kühlelement 321 als Durchlaufkühler ausgelegt. Des weiteren können auch Peltier-Elemente in den Kühlelementen 321, 322, 323, 324 verwendet werden.

Falls erforderlich, kann das zweite Kühlvolumen 32 auch durch nur drei, zwei oder durch ein einziges dieser Kühlelemente 321, 322, 323, 324 gebildet sein, insb. dann, wenn wie in der Figur dargestellt, das zweite Kühlvolumen 32 bevorzugt wenigstens teilweise vom ersten Kühlvolumen 31 umschlossen wird.

Vorteilhaft bei dieser Ausgestaltung ist, daß bereits bestehende Probenehmer ohne weiteres mit derartigen Kühlelementen nachrüstbar sind.

Nach einer weiteren Ausgestaltung des Verfahrens der Erfindung wird die Gefäßanordnungs-Innentemperatur T₂ vor der Entnahme des ersten Fluid-Volumens mittels eines oder mehrerer der Kühlelemente 321, 322, 323 bzw. 324 temporär auf die Abkühlungs-Gefäßanordnungs-Innentemperatur von z.B. ebenfalls 273 K, abgesenkt, so daß die Gefäßanordnungs-Innentemperatur T₂, insb. zum Zeitpunkt t₁, wiederum niedriger als die Anfangs-Gefäßanordnungs-Innentemperatur ist.

Während oder nach dem Absenken der zweiten Kühltemperatur T₃₂ wird das Fluid wiederum in die Gefäßanordnung 2 einströmen gelassen und dadurch auf eine Fluid-Temperatur abgekühlt, die unterhalb der Entnahme-Temperatur T₁ liegt. Selbstverständlich kann bei dieser Ausgestaltung der Erfindung auch das zuvor beschriebene Verfahren, bei dem die erste Kühltemperatur T₃₁ abgesenkt wird, mit zur Anwendung kommen.

Vor dem Beginn des Entnahmebetriebes werden üblicherweise, insb. bei mehreren aufeinanderfolgenden Abfüllvorgängen, fluidische Rückstände aus der Gefäßanordnung 2 entfernt. Dies erfolgt in geeigneter Weise z.B. dadurch, daß bei verschlossener Auslaß-Öffnung des Dosiergefäßes 22 und bei gleichzeitig geöffneter Ein/Auslaß-Öffnung mittels der Druck-Quelle 5 ein statischer Überdruck im Lumen des Dosiergefäßes 22 und des angeschlossenen Entnahmegefäßes 21 erzeugt wird. Aufgrund dieses Überdruckes werden im Entnahmegefäß 21 befindliche Rückstände durch dessen erstes Ende herausdrückt. Falls erforderlich, kann außerdem durch ein Verschließen der Ein/Auslaß-Öffnung bei gleichzeitig geöffneter Auslaß-Öffnung mittels eines im Lumen des Dosiergefäßes 22 erzeugten Überdrucks das Dosiergefäß selbst sowie das angeschlossene Verteilergefäß 23 von fluidischen Rückständen befreit werden; letztere können dann über das zweite Ende des Verteilergefäßes 23 in ein geeignetes Gefäß des Probenehmers oder aus diesem heraus geleitet werden.

## Patentansprüche

1. Probenehmer zum Abfüllen und Abkühlen einer bei einer vorgebbaren Lagerungs-Temperatur einzulagernden Fluid-Probe eines an einer Fluid-Entnahmestelle (1) entnommenen Fluids, das eine momentane Entnahme-Temperatur aufweist, die größer als die Lagerungs-Temperatur ist, welcher Probenehmer umfaßt:
- eine Gefäßanordnung (2) von vorgebbarem Lumen mit
-- einer über das Lumen räumlich gemittelten Gefäßanordnungs-Innentemperatur
-- einem rohrförmigen Entnahmegefäß (21) zum Führen eines strömenden Fluids,
-- einem auslaßseits temporär verschließbaren Dosiergefäß (22) zum Abdosieren der Fluid-Probe vom entnommenen Fluid,
-- einem Verteilergefäß (23) zum Weiterleiten der Fluid-Probe und
-- einem Speichergefäß (24) zum Speichern der Fluid-Probe sowie
- eine mit der Gefäßanordnung (2) thermisch gekoppelte Kühlanordnung (3) zum Einstellen der Gefäßanordnungs-Innentemperatur, mit
-- einem wenigstens das Speichergefäß (24) umhüllenden ersten Kühlvolumen von vorgebbarer erster Kühltemperatur zum Kühlen der Fluid-Probe auf die Lagerungs-Temperatur **dadurch gekennzeichnet, daß** der Probenehmer
-- ein die Gefäßanordnung (2) wenigstens abschnittsweise ummantelndes zweites Kühlvolumen von vorgebbarer zweiter Kühltemperatur zum Kühlen des entnommenen Fluids unter die Fluid-Entnahmetemperatur umfaßt.

2. Probenehmer nach Anspruch 1, bei dem das zweite Kühlvolumen vom ersten Kühlvolumen wenigstens teilweise umhüllt ist.

3. Probenehmer nach Anspruch 1 oder 2, bei dem die Kühlanordnung (3) umfaßt:
- ein am Entnahmegefäß (21) angeordnetes erstes Kühlelement (321) zum Einstellen einer Entnahmegefäß-Innentemperatur und/oder
- ein am Dosiergefäß (22) angeordnetes zweites Kühlelement (322) zum Einstellen einer Dosiergefäß-Innentemperatur und/oder
- ein am Verteilergefäß (23) angeordnetes drittes Kühlelement (323) zum Einstellen einer Speichergefäß-Innentemperatur und/oder
- ein am Speichergefäß (24) angeordnetes viertes Kühlelement (324) zum Einstellen der Speichergefäß-Innentemperatur,
- wobei das jeweilige Kühlelement (321, 322, 323, 324) das zweite Kühlvolumen abschnittsweise ummantelt.

4. Probenehmer nach Anspruch 3, bei dem das erste Kühlelement (321) ein Durchlaufkühler ist.

5. Probenehmer nach Anspruch 3, bei dem wenigstens eines der Kühlelemente (321, 322, 323, 324) ein Peltier-Element umfaßt.

6. Probenehmer nach einem der Ansprüche 1 bis 5, bei dem das Dosiergefäß (22) innerhalb des ersten Kühlvolumens angeordnet ist.

7. Probenehmer nach Anspruch 6, bei dem das Entnahmegefäß (21) vom ersten Kühlvolumen teilweise umhüllt ist.

8. Probenehmer nach einem der Ansprüche 1 bis 7, bei dem die Kühlanordnung (3) einen Kühlraum (311) umfaßt, dessen Lumen das erste Kühlvolumen ist.

9. Probenehmer nach einem der Ansprüche 1 bis 8, bei dem das Fluid ein Trinkwasser oder ein Abwasser ist.

10. Verfahren zum Abfüllen und Abkühlen einer bei einer vorgebbaren Lagerungs-Temperatur einzulagernden Fluid-Probe eines an einer Fluid-Entnahmestelle (1) entnommenen Fluids, das eine momentane Entnahme-Temperatur aufweist, die größer als die Lagerungs-Temperatur ist, mittels eines Probenehmers, der umfaßt:
- eine Gefäßanordnung (2) von vorgebbarem Lumen mit
-- einer über das Lumen räumlich gemittelten Gefäßanordnungs-Innentemperatur
-- einem rohrförmigen Entnahmegefäß (21) zum Führen eines strömenden Fluids,
-- einem auslaßseits temporär verschließbaren Dosiergefäß (22) zum Abdosieren der Fluid-Probe vom entnommenen Fluid,
-- einem Verteilergefäß (23) zum Weiterleiten der Fluid-Probe,
-- mit einem Speichergefäß (24) zum Speichern der Fluid-Probe, und
- eine mit der Gefäßanordnung (2) thermisch gekoppelte Kühlanordnung (3) zum Einstellen der Gefäßanordnungs-Innentemperatur mit
-- einem wenigstens das Speichergefäß (24) umhüllenden ersten Kühlvolumen von vorgebbarer erster Kühltemperatur zum Kühlen der Fluid-Probe auf die Lagerungs-Temperatur, wobei
- die Gefäßanordnungs-Innentemperatur vor dem Abfüllen eine Anfangs-Gefäßanordnungs-Innentemperatur aufweist, die niedriger als die Entnahme-Temperatur ist, und
- die erste Kühltemperatur vor dem Abfüllen und nach dem Einlagern der Fluid-Probe auf die Lagerungs-Temperatur eingestellt ist,
welches Verfahren folgende Schritte umfaßt:
- die Fluid-Probe wird dadurch abgefüllt, daß
-- das Fluid von der Fluid-Entnahmestelle (1) aus durch das Entnahmegefäß (21) hindurch in das auslaßseits verschlossene Dosiergefäß (22) gefördert wird,
-- ein vorgebbares Teil-Volumen des Fluids im Dosiergefäß (22) belassen wird,
-- das Dosiergefäß (22) auslaßseits geöffnet wird und
-- das Teil-Volumen des Fluids durch das Verteilergefäß (23) hindurch in das Speichergefäß (24) einströmen gelassen wird, **dadurch gekennzeichnet, daß**
- die Fluid-Probe dadurch abgekühlt wird daß
-- die Gefäßanordnungs-Innentemperatur mittels der Kühlanordnung (3) vor und/oder während des Abfüllens auf eine Abkühlungs-Gefäßanordnungs-Innentemperatur abgesenkt wird, die niedriger als die Anfangs-Gefäßanordnungs-Innentemperatur ist, und
-- das Fluid nach dem Einstellen der Abkühlungs-Gefäßanordnungs-Innentemperatur in der Gefäßanordnung (2) geführt und/oder gehalten wird.

11. Verfahren nach Anspruch 10, bei dem die Gefäßanordnungs-Innentemperatur dadurch auf die Abkühlungs-Gefäßanordnungs-Innentemperatur eingestellt wird, daß die erste Kühltemperatur temporär auf eine erste Abkühlungs-Temperatur abgesenkt wird, die niedriger als die Lagerungs-Temperatur ist.

12. Verfahren nach Anspruch 10 oder 11,
- bei dem eine Kühlanordnung (3) mit einem die Gefäßanordnung (2) wenigstens abschnittsweise umgebenden zweiten Kühlvolumen von vorgebbarer zweiter Kühltemperatur zum Kühlen des entnommenen Fluids unter die Entnahme-Temperatur verwendet wird und
- bei dem die Abkühlungs-Gefäßanordnungs-Innentemperatur vor dem Einströmen des Fluids in das Dosiergefäß (22) durch ein temporäres Einstellen der zweiten Kühltemperatur auf eine zweite Abkühlungs-Temperatur, die niedriger als die Lagerungs-Temperatur ist, eingestellt wird.

13. Verfahren nach einem der Ansprüche 10 bis 12, bei dem die Fluid-Probe von einem wäßriges Fluid entnommen wird.

## Claims

1. A sampler for drawing off and cooling down a fluid sample, to be stored at a specifiable storage temperature, of a fluid extracted at a fluid sampling point (1), which has an instantaneous sampling temperature which is greater than the storage temperature, which sampler comprises:
- a vessel arrangement (2) of specifiable lumen with
-- a vessel arrangement internal temperature obtained spatially over the lumen,
-- a tubular sampling vessel (21) for conducting a flowing fluid,
-- a metering vessel (22), temporarily closable on the outlet side, for metering out the fluid sample from the extracted fluid,
-- a distributing vessel (23) for passing on the fluid sample, and
-- a storage vessel (24) for storing the fluid sample, and also
- a cooling arrangement (3) thermally coupled with the vessel arrangement (2) for adjusting the vessel arrangement internal temperature, with
-- a first cooling volume of specifiable first cooling temperature, enclosing at least the storage vessel (24), for cooling the fluid sample to the storage temperature, **characterised in that** the sampler comprises
-- a second volume of specifiable second cooling temperature, encasing the vessel arrangement (2) at least in parts, for cooling the extracted fluid below the fluid sampling temperature.

2. A sampler according to Claim 1, in which the second cooling volume is enclosed at least partially by the first cooling volume.

3. A sampler according to Claim 1 or 2, in which the cooling arrangement (3) comprises:
- a first cooling element (321), arranged on the sampling vessel (21), for adjusting a sampling vessel internal temperature, and/or
- a second cooling element (322), arranged on the metering vessel (22), for adjusting a metering vessel internal temperature, and/or
- a third cooling element (323), arranged on the distributing vessel (23), for adjusting a storage vessel internal temperature, and/or
- a fourth cooling element (324), arranged on the storage vessel (24), for adjusting the storage vessel internal temperature,
- wherein the respective cooling element (321, 322, 323, 324) encases the second cooling volume in parts.

4. A sampler according to Claim 3, in which the first cooling element (321) is a continuous-flow cooler.

5. A sampler according to Claim 3, in which at least one of the cooling elements (321, 322, 323, 324) comprises a Peltier element.

6. A sampler according to Claims 1 to 5, in which the metering vessel (22) is arranged within the first cooling volume.

7. A sampler according to Claim 6, in which the sampling vessel (21) is partially enclosed by the first cooling volume.

8. A sampler according to one of Claims 1 to 7, in which the cooling arrangement (3) includes a cooling chamber (311), the lumen of which is the first cooling volume.

9. A sampler according to one of Claims 1 to 8, in which the fluid is a drinking water or a waste water.

10. A method for drawing off and cooling down a fluid sample, to be stored at a specifiable storage temperature, of a fluid extracted at a fluid sampling point (1), which has an instantaneous sampling temperature which is greater than the storage temperature, by means of a sampler which comprises:
- a vessel arrangement (2) of specifiable lumen with
-- a vessel arrangement internal temperature obtained spatially over the lumen,
-- a tubular sampling vessel (21) for conducting a flowing fluid,
-- a metering vessel (22), temporarily closable on the outlet side, for metering out the fluid sample from the extracted fluid,
-- a distributing vessel (23) for passing on the fluid sample,
-- with a storage vessel (24) for storing the fluid sample, and
- a cooling arrangement (3) thermally coupled with the vessel arrangement (2) for adjusting the vessel arrangement internal temperature, with
-- a first cooling volume of specifiable first cooling temperature, enclosing at least the storage vessel (24), for cooling the fluid sample to the storage temperature, wherein
- the vessel arrangement internal temperature before the drawing-off has an initial vessel arrangement internal temperature which is lower than the sampling temperature, and
- the first cooling temperature before the drawing-off and after the storing of the fluid sample is adjusted to the storage temperature,
which method comprises the following steps:
- the fluid sample is drawn off by
-- the fluid being transferred from the fluid sampling point (1) through the sampling vessel (21) into the metering vessel (22) which is closed on the outlet side,
-- a specifiable part-volume of the fluid being left in the metering vessel (22),
-- the metering vessel (22) being opened on the outlet side, and
-- the part-volume of the fluid being allowed to flow through the distributing vessel (23) into the storage vessel (24), **characterised in that**
- the fluid sample is cooled down by
-- the vessel arrangement internal temperature being lowered by means of the cooling arrangement (3) before and/or during the drawing-off to a cooling-down vessel arrangement internal temperature which is lower than the initial vessel arrangement internal temperature, and
-- the fluid being conducted and/or held in the vessel arrangement (2) after the adjusting of the cooling-down vessel arrangement internal temperature.

11. A method according to Claim 10, in which the vessel arrangement internal temperature is adjusted to the cooling-down vessel arrangement internal temperature by the first cooling temperature being temporarily lowered to a first cooling-down temperature which is lower than the storage temperature.

12. A method according to Claim 10 or 11,
- in which a cooling arrangement (3) with a second cooling volume of specifiable second cooling temperature, enclosing the vessel arrangement (2) at least in parts, is used to cool the extracted fluid below the sampling temperature, and
- in which the cooling-down vessel arrangement internal temperature prior to the inflow of the fluid into the metering vessel (22) is adjusted by means of a temporary adjustment of the second cooling temperature to a second cooling-down temperature which is lower than the storage temperature.

13. A method according to one of Claims 10 to 12, in which the fluid sample is extracted from an aqueous fluid.

## Revendications

1. Echantillonneur destiné à verser et à refroidir un échantillon de fluide à stocker avec une température de stockage prédéterminée d'un fluide retiré à l'endroit de retrait de fluide (1), lequel fluide présente une température de retrait momentanée supérieure à la température de stockage , l' échantillonneur comportant :
- un dispositif à récipient (2) avec un lumen susceptible d'être prédéterminé avec
■ une température intérieure moyennée de façon tridimensionnelle sur le lumen du dispositif de récipients,
■ un récipient de retrait (21) tubulaire pour guider un fluide affluant,
■ un récipient de dosage (22) susceptible d'être temporairement fermé du côté de la sortie pour doser l'échantillon de fluide du fluide retiré,
■ un récipient de répartition (23) pour transférer l'échantillon de fluide et
■ un récipient de stockage (24) pour stocker l'échantillon de fluide ainsi qu'
un dispositif de refroidissement (3) couplé thermiquement avec le dispositif de récipient (2), lequel dispositif de refroidissement est destiné à régler la température intérieure du dispositif à récipient, avec
■ un premier volume de refroidissement entourant au moins le récipient de stockage (24) , avec une première température de refroidissement susceptible d'être prédéterminée pour refroidir l'échantillon de fluide à la température de stockage **caractérisé en ce que** l'échantillonneur comporte
■ un deuxième volume de refroidissement entourant au moins par sections le dispositif à récipient (2) , avec une deuxième température de refroidissement susceptible d'être prédéterminée pour refroidir le fluide retiré sous la température de retrait de fluide.

2. Echantillonneur selon la revendication 1, dans lequel le deuxième volume de refroidissement est entouré au moins partiellement par le premier volume de refroidissement.

3. Echantillonneur selon la revendication 1 ou 2, dans lequel le dispositif de refroidissement (3) comporte :
- un premier élément de refroidissement (321) disposé sur le récipient de retrait (21) pour régler une température intérieure de récipient de retrait et/ou
- un deuxième élément de refroidissement (322) disposé sur le récipient de dosage (22) pour régler une température intérieure de récipient de dosage et / ou
- un troisième élément de refroidissement (323) disposé sur le récipient de répartition (23) pour régler une température intérieure de récipient de stockage et / ou
- un quatrième élément de refroidissement (324) disposé sur le récipient de stockage (24) pour régler une température intérieure de récipient de stockage
- où l'élément de refroidissement respectif (321, 322, 323, 324) entoure par sections le deuxième volume de refroidissement.

4. Echantillonneur selon la revendication 3, dans lequel le premier élément de refroidissement (321) est un refroidisseur de flux.

5. Echantillonneur selon la revendication 3, dans lequel au moins un des éléments de refroidissement (321, 322, 323, 324) est un élément de Peltier.

6. Echantillonneur selon une des revendications 1 à 5, dans lequel le récipient de dosage (22) est disposé à l'intérieur du premier volume de refroidissement.

7. Echantillonneur selon la revendication 6, dans lequel le récipient de retrait (21) est partiellement enveloppé par le premier volume de refroidissement.

8. Echantillonneur selon une des revendications 1 à 7, dans lequel le dispositif de refroidissement (3) comporte une chambre de refroidissement (311), dont le lumen est le premier volume de refroidissement.

9. Echantillonneur selon une des revendications 1 à 8, dans lequel le fluide est un eau potable ou un eau usée.

10. Procédé pour verser et refroidir, au moyen d'un échantillonneur, un échantillon de fluide à stocker avec une température de stockage prédéterminée d'un fluide retiré à l'endroit de retrait de fluide (1) momentané, lequel fluide présente une température de retrait momentanée supérieure à la température de stockage, l'échantillonneur comportant :
- un dispositif à récipients (2) avec un lumen susceptible d'être prédéterminé avec
■ une température intérieure moyennée de façon tridimensionnelle sur le lumen du dispositif à récipients,
■ un récipient de retrait (21) tubulaire pour guider un fluide affluant,
■ un récipient de dosage (22) susceptible d'être temporairement fermé du côté de la sortie pour doser l'échantillon de fluide du fluide retiré,
■ un récipient de répartition (23) pour transférer l'échantillon de fluide et
■ un récipient de stockage (24) pour stocker l'échantillon de fluide ainsi qu'
un dispositif de refroidissement (3) couplé thermiquement avec le dispositif à récipient (2) , lequel dispositif de refroidissement étant destiné à régler la température intérieure du dispositif à récipient, avec
- un premier volume de refroidissement entourant au moins le récipient de stockage (24) , avec une première température de refroidissement susceptible d'être prédéterminée pour refroidir l'échantillon de fluide à la température de stockage, où
- la température intérieure du dispositif de récipient présente avant le versement, une température intérieure de dispositif de récipients de départ inférieure à la température de retrait, et
- la première température de refroidissement est réglée sur la température de stockage avant le versement et après le stockage de l'échantillon de fluide,
lequel procédé comporte les étapes suivantes :
- l'échantillon de fluide est versé en
■ transportant le fluide à partir de l'endroit de retrait (1) de fluide à travers le récipient de retrait (21) dans le récipient de dosage (22) fermé du côté de la sortie,
■ un volume partiel prédéterminé de fluide reste dans le récipient de dosage (22) ,
■ le récipient de dosage (22) est ouvert du côté de la sortie et
■ le volume partiel du fluide rentre à travers le récipient de répartition dans le récipient de stockage, **caractérisé en ce que**
- l'échantillon de fluide est refroidi en
■ baissant la température intérieure du dispositif de récipients au moyen du dispositif de refroidissement avant et / ou pendant le remplissage à une température intérieure du dispositif de récipients de refroidissement inférieure à la température intérieure à la température intérieure du dispositif de récipients, et
■ en guidant et / ou en maintenant le fluide après le réglage de la température intérieure du dispositif de récipients de refroidissement dans le dispositif à récipient (2).

11. Procédé selon la revendication 10, dans lequel la température intérieure du dispositif de récipients est réglée à la température intérieure du dispositif de récipient de refroidissement en baissant temporairement la première température de refroidissement à la première température de refroidissement, qui est inférieure à la température de stockage.

12. Procédé selon la revendication 10 ou 11 ,
- dans lequel on utilise un dispositif de refroidissement avec un deuxième volume de refroidissement entourant au moins par sections le dispositif de récipients et qui est pourvu d'une deuxième température de refroidissement prédéterminée pour refroidir le fluide retiré à la température de retrait et
- dans lequel on règle la température intérieure de dispositif de récipients de refroidissement avant l'afflux du fluide dans le récipient de dosage au moyen d'un réglage temporaire de la deuxième température de refroidissement à la deuxième température de refroidissement qui est inférieure à la température de stockage.

13. Procédé selon une des revendications 10 à 12, dans lequel l'échantillon de fluide est retiré d'un fluide aqueux.
